# DEMANDE DE BREVET EUROPEEN

(11) **EP 0 729 759 A1**
(43) Date de publication de la demande: **04.09.1996**
(21) Numéro de dépôt: 96420066.1
(22) Date de dépôt: 29.02.1996
(51) Int. Cl.: A61L 2/26, A01N 1/02

(54) **Conditionnement de stérilisation et de conservation de matières organiques**

(30) Priorité: 01.03.1995 FR 9502655
(71) Demandeur: MERCK BIOMATERIAL FRANCE, F-01800 Charnoz (FR); Chiron, Philippe Charles, 31100 Toulouse (FR)
(72) Inventeur: Chiron, Philippe Charles, 31100 Toulouse (FR); Collomb, Jean, 26800 Portes Les Valence (FR); Picault, Charles, 69110 Sainte-Foy-Les-Lyon (FR)
(74) Mandataire: Ropital-Bonvarlet, Claude

(57) **Abrégé**

- Stérilisation de matières organiques.
- Le conditionnement comprend un récipient **(1)** et une coiffe **(7)**, ce récipient étant réalisé en une matière au moins translucide et les moyens **(20)** établis à distance du fond étant prévus pour
   - délimiter un logement **(24)** de réception et de maintien de la matière organique, un tel logement étant ouvert à l'opposé du compartiment **(21)** et communiquant avec ce dernier,
   - déterminer au moins un niveau de remplissage du compartiment,
   - et maintenir ladite matière à distance de la face interne de la paroi périphérique **(3)** du récipient.
- Application à la stérilisation et à la conservation de transplants osseux.

## Description

La présente invention est relative au domaine de la conservation de matières et de matériels organiques, en vue de permettre leur utilisation ultérieure en toute sécurité.

L'invention peut concerner différents domaines d'application, tels que celui de la conservation de produits alimentaires ou de la conservation de matériels organiques réutilisables dans le domaine pharmaceutique, médical, biologique etc.

La conservation de matières ou matériels organiques a déjà fait l'objet de publications relativement nombreuses faisant état de méthodes de conservation variées.

Il peut être cité, à titre indicatif, l'incubation telle qu'enseignée par la demande EP-0.584.484, la cryoconservation, la lyophilisation, ainsi que la stérilisation.

La première méthode n'apparaît pas de nature à satisfaire les exigences de suppression des risques de contamination résiduelle de la matière.

Les deux méthodes suivantes présentent l'inconvénient de faire intervenir des procédés lourds et relativement coûteux qui, par ailleurs, ne fournissent pas une sécurité absolue quant aux risques de contamination résiduelle par des microorganismes ou par des virus.

Le procédé de stérilisation peut connaître différentes méthodes, telles que l'application de rayonnements, l'application d'oxyde d'éthylène ou la stérilisation par chaleur humide.

L'application de rayonnements, du type X, Gamma ou bêta, implique un matériel important et de conduite délicate. Par ailleurs, de tels rayonnements sont à l'origine d'une altération des propriétés des matières organiques dont la réutilisation peut être de la sorte, en partie gênée, voire entravée. De plus, le coût de mise en oeuvre d'une telle méthode est élevé.

La stérilisation par l'oxyde d'éthylène possède l'inconvénient de représenter pour le personnel un risque, soit direct, soit par l'intermédiaire des produits de réaction secondaire, tels que l'éthylène-glycol et l'éthylène-chloridrine, produits réputés toxiques.

La stérilisation par chaleur humide apparaît une technique intéressante, en raison de la possibilité qu'elle offre d'être menée à bien, à partir de moyens techniques relativement simples, avec une dépense d'énergie relativement faible.

Le problème qui se pose toutefois pour la mise en oeuvre d'une telle méthode est de disposer d'un matériel qui permette de mener à bien le processus de stérilisation par voie humide, tel qu'il est connu, au moyen d'un autoclave et d'un processus d'application séquentielle de températures bien déterminées et qui permette, de surcroît, d'assurer une conservation de la matière ou du matériel stérilisé dans des conditions de sécurité absolue rendant possible un stockage, un transport, ainsi qu'une possible réutilisation dans des conditions optimales.

Un autre problème est celui de préserver la matière ou le matériel organique devant être stérilisé, en étant assuré visuellement que la stérilisation intervenue correspond exactement au processus opératoire ayant été conduit.

Il peut être estimé qu'à l'heure actuelle, la technique connue ne fournit pas de matériel propre à répondre aux exigences ci-dessus.

L'objet de l'invention est de combler la présente lacune en proposant un conditionnement de stérilisation et de conservation qui répondent aux exigences ci-dessus, tout en étant d'un prix de revient intéressant et d'une manipulation simple n'exigeant pas de qualification particulière pour sa mise en oeuvre.

Pour atteindre les objectifs ci-dessus, le conditionnement de stérilisation et de conservation de matière organique conforme à l'invention, du type comprenant un récipient ou analogue pourvu d'un fond, des moyens établis à distance du fond pour délimiter un compartiment au moins en partie ouvert et pour supporter la matière organique, et une coiffe à même de fermer le récipient de façon étanche, est caractérisé en ce que le récipient est réalisé en une matière au moins translucide et en ce que des moyens, établis à distance du fond, sont prévus pour
- délimiter un logement de réception et de maintien de la matière organique, un tel logement étant ouvert à l'opposé du compartiment et communiquant avec ce dernier,
- déterminer au moins un niveau de remplissage du compartiment,
- et maintenir ladite matière à distance de la face interne de la paroi périphérique du récipient.

Diverses autres caractéristiques ressortent de la description faite ci-dessous en référence aux dessins annexés qui montrent, à titre d'exemples non limitatifs, des formes de réalisation de l'objet de l'invention.

La figure **1** est une coupe-élévation du conditionnement de stérilisation et de conservation conforme à l'invention.

La figure **2** est une coupe-élévation partielle montrant, à plus grande échelle, un détail constructif des moyens de l'invention.

Les figures **3** et **4** sont des perspectives illustrant deux variantes de construction de l'un des éléments constitutifs du conditionnement.

La figure **5** est une perspective, partie en coupe, montrant une autre variante de réalisation.

La figure **6** représente une coupe-élévation illustrant un autre mode de construction de l'objet de l'invention.

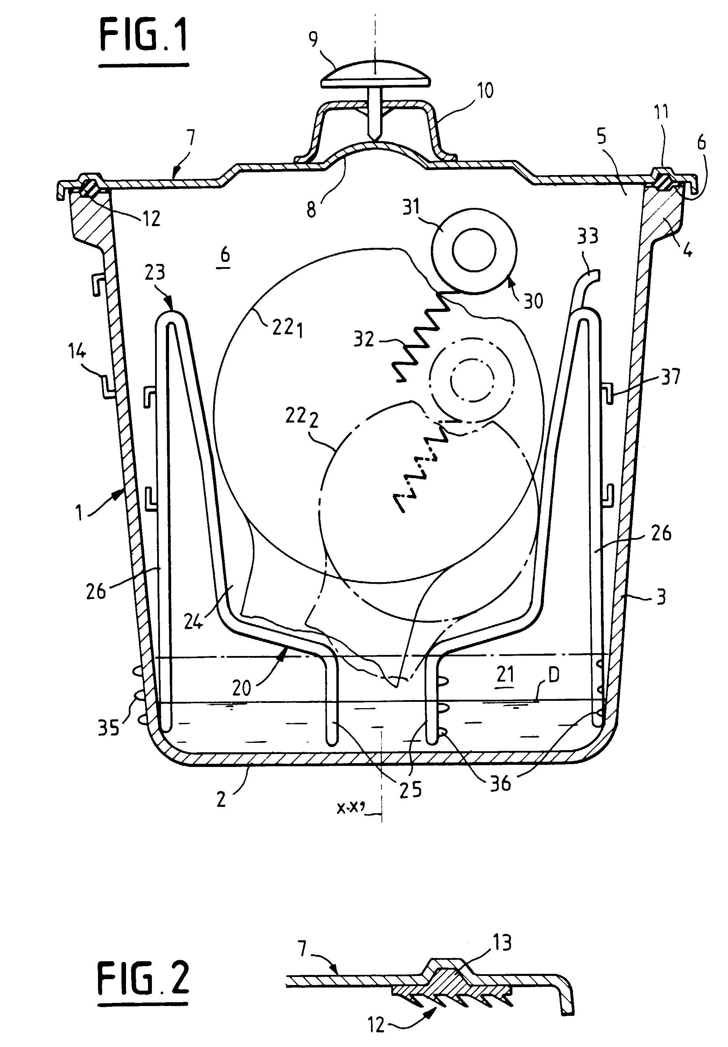

Selon la figure **1**, le conditionnement de stérilisation et de conservation conforme à l'invention comprend un récipient **1** ou analogue défini par un fond **2** bordé par une paroi périphérique **3** qui peut être de conformation cylindrique, cylindro-tronconique ou tronconique. Généralement, la paroi périphérique **3** est de forme tronconique de conicité s'ouvrant vers le haut et comporte un bord **4** supérieur concentrique à un axe de révolution **xx'** et définissant une ouverture **5** d'accès au volume interne **6** que définit le récipient **1**.

Au sens de l'invention, le récipient **1**, ou tout au moins la paroi périphérique **3**, est réalisé en un matériau au moins translucide, de préférence transparent. Le récipient **1** peut ainsi être réalisé entièrement en/ou pour partie en verre ou en une matière plastique choisie alors parmi celles à même de supporter sans dommage les contraintes mécaniques et thermiques résultant d'une opération de stérilisation menée dans les conditions habituelles connues.

Le récipient **1**, dans la forme de réalisation représentée à la figure **1**, s'apparente à un bocal dont le bord supérieur **4** délimite une plage **6** permettant l'application d'une coiffe **7** à même de fermer de façon étanche le volume **6**. La coiffe **7** est réalisée sous la forme d'une capsule métallique portant, sur sa face extérieure et par exemple en relation avec un bulbe **8**, un perforateur **9** monté dans un pontet **10** et susceptible d'être actionné manuellement, à partir d'une position d'effacement ou de retrait illustrée à la figure **1**, pour être amené à perforer le bulbe **8**, lorsque les conditions d'ouverture, telles qu'elles ressortent de ce qui suit, deviennent nécessaires.

La figure **1** doit être considérée comme fournissant un exemple de réalisation pouvant admettre des variantes de construction. C'est ainsi que le récipient **1** pourrait présenter une paroi périphérique de hauteur notablement inférieure et que la coiffe **7** pourrait alors être constituée sous la forme d'un bocal renversé.

Dans tous les cas, la coiffe **7** comporte, dans une feuillure annulaire 11, une garniture d'étanchéité **12** à même de coopérer avec la plage **6**, lors de la phase de stérilisation et de mise sous vide du volume **6**. Selon la figure **2**, la garniture d'étanchéité **12** est constituée sous la forme d'un joint **13** à lèvres multiples qui peuvent être naturellement orientées, de préférence selon une direction inclinée vers le bord périphérique de la coiffe **7**.

La figure 1 montre qu'il est avantageux de prévoir, en surface extérieure de la paroi périphérique **3**, des moyens **14** de montage, de présentation et de visualisation d'informations d'identification.

Au sens de l'invention, le conditionnement de stérilisation et de conservation comprend des moyens **20** établis à distance du fond **2** pour assumer différentes fonctions spécifiques.

La première de ces fonctions est de ménager un compartiment **21** à l'intérieur du volume **6**, un tel compartiment étant délimité à partir du fond 2 et présentant une ouverture de communication avec le volume **6**.

La deuxième fonction est de supporter la matière organique, telle que **22,** devant être stérilisée et conservée à l'intérieur du récipient.

La troisième fonction est d'assurer la protection de la matière **22** dans le but, notamment, de lui éviter des contacts ponctuels, plus ou moins intenses et brutaux, avec la paroi **3** lors des phases de manipulation.

Pour assumer les trois fonctions ci-dessus, les moyens **20**, dans une forme de réalisation représentée à la figure **1**, sont constitués sous la forme d'un support ajouré **23** amovible, disposé à l'intérieur du récipient **1** en prenant appui sur le fond **2**. Le support ajouré **23** peut être constitué sous la forme d'une sorte de gobelet, par exemple formé par la réunion de fils ou tiges qui sont associés par moulage ou encore par tout autre mode de liaison. Le gobelet **23** pourrait aussi être réalisé sous la forme d'une enveloppe perforée.

Le gobelet délimite un logement **24** qui est ouvert vers le haut pour permettre l'insertion, le dépôt ou l'introduction de la matière **22** et qui est ajouré, perforé ou ouvert vers le bas pour communiquer avec le compartiment **21**. Le gobelet **23** est pourvu de pattes **25** s'étendant vers le bas pour prendre appui sur le fond **2** et ménager ainsi le compartiment **21**.

Le gobelet **23** comporte aussi avantageusement des barrettes **26** s'étendant vers le bas à partir du dessus ouvert, en étant disposées à l'extérieur pour coopérer avec le fond **2** et/ou la paroi périphérique **3**. Les barrettes **26** contribuent ainsi au support du gobelet et maintiennent ce dernier à distance de la paroi **3**.

Dans une application préférée, mais non limitative de l'invention, le conditionnement de stérilisation et de conservation est conçu pour assurer la stérilisation et la conservation de matériels organiques et, de préférence encore, de transplants osseux qui sont, à titre d'exemple, illustrés à la figure **1** sous la forme de têtes de fémur.

Les deux représentations désignées par les références **22**_{**1**} et **22**_{**2**} permettent de constater que le gobelet **23** est à même d'assurer les fonctions rappelées ci-dessus pour différentes tailles de têtes de fémur qui peuvent subir les phases de stérilisation habituelles, en étant maintenues dans un état de conservation optimale, en raison de la présence du compartiment **21** qui assume deux fonctions.

La première fonction est celle de réservoir destiné à contenir une dose **D** de liquide vaporisable, tel que du sérum physiologique, produisant une vapeur humide saturée lors de la phase de stérilisation.

La seconde est celle de réception des exsudats organiques qui peuvent être produits lors de la phase de stérilisation. De tels exsudats, lors qu'ils sont produits, se déposent dans le compartiment **21**, ce qui évite la pollution par dépôt sur la matière ou le matériel **22.**

Selon une autre disposition, le gobelet **23** est associé à des moyens de préhension **30** qui peuvent être utilisés pour introduire, déposer ou placer le matériel **22** dans le logement **24**, mais aussi pour permettre un prélèvement stérile lors de l'utilisation de cette matière ou de ce matériel. Les moyens de préhension peuvent consister en un anneau **31** ouvert ou fermé, prolongé par un crochet ou une tige spiralée **32**. Il peut être envisagé de munir le gobelet **23** d'un picot ou d'un doigt **33** engageable avec le moyen **30** pour que ce dernier assume une fonction supplémentaire d'immobilisation de la matière ou du matériel dans le logement **24**.

L'utilisation du conditionnement, tel que décrit ci-dessus, consiste à placer le gobelet **23** à l'intérieur du récipient **1**, puis à disposer dans le compartiment **21** la dose **D** de produit liquide nécessaire pour mener à bien le procédé de stérilisation par voie humide.

A cet égard, il est avantageux au sens de l'invention, de prévoir des moyens de jaugeage d'un tel niveau. De tels moyens peuvent être portés par la paroi périphérique **3** où ils sont désignés par la référence **35** ou encore par des graduations telles que **36** portées par les pattes **25** ou encore par les barrettes **26**.

Par l'intermédiaire du ou des moyens de préhension **30**, la matière ou le matériel **22** est déposé dans le logement **24**, puis la coiffe **7** est placée sur le rebord **4** sur lequel elle est maintenue par tout moyen approprié, avant de disposer le conditionnement dans un autoclave permettant de mener à bien le processus de stérilisation.

Un tel processus est largement connu de la technique et n'a pas à être évoqué davantage. Il suffit de préciser qu'au cours de ce processus, un vide s'établit à l'intérieur du récipient **1**, puis une montée en température produisant la vaporisation de la dose **D** de sérum physiologique par exemple.

Tous ces facteurs de conduite sont maintenus pendant une durée, par exemple égale à 20 minutes, avec une température de 120° C pour assurer la stérilisation de la matière ou du matériel **22** qui reste confiné de façon étanche à l'intérieur du volume **6** et du logement **24** par la fermeture étanche de la coiffe **7**.

Dans cet état, tel qu'illustré par la figure **1**, et après refroidissement, le conditionnement ayant permis la stérilisation est à même d'assumer la fonction de conservation de la matière ou du matériel **22,** tout en permettant de surcroît une manipulation pour favoriser les stockages, les transports etc. Pendant toute la durée de conservation et lors de l'utilisation, le conditionnement permet une lecture des moyens d'identification, ainsi qu'une appréciation visuelle de l'état stérilisé de la matière.

Lorsqu'il convient d'utiliser la matière **22,** il suffit de perforer la coiffe **7** par l'intermédiaire du perforateur **9**, pour rétablir à l'intérieur du volume **6** la pression atmosphérique et permettre ainsi l'ouverture de la coiffe **7**.

La matière **22** peut alors être prélevée et extraite du récipient **1**, par l'intermédiaire de l'un ou des organes de préhension **30**.

Il est avantageux aussi de faire porter au support **23** des moyens **37** d'adaptation de témoin de stérilisation et/ou d'information d'identification de la matière **22.**

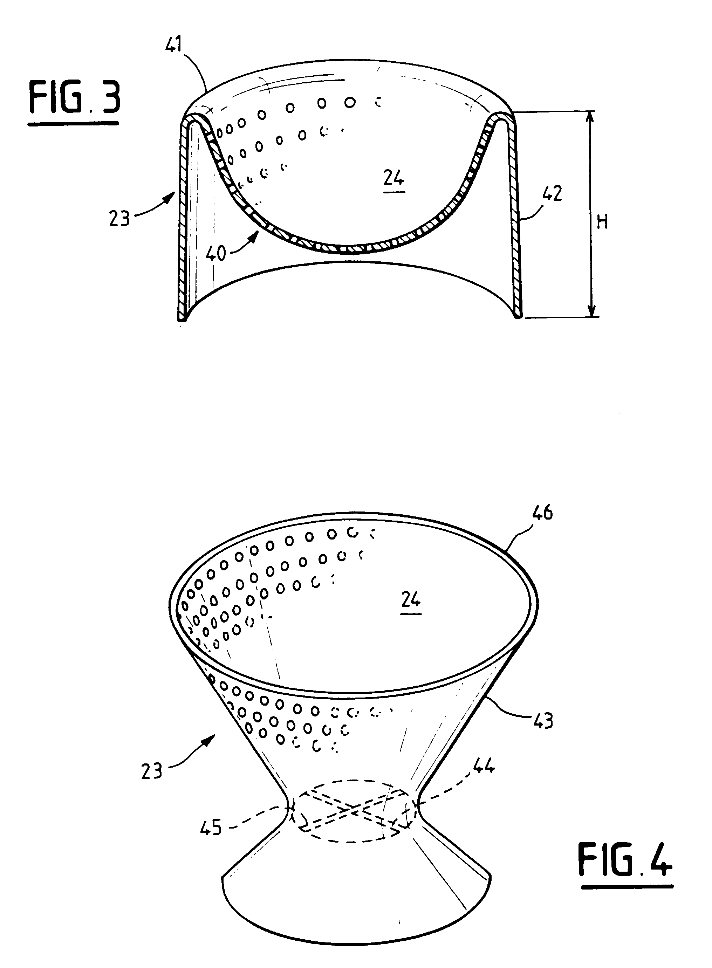

La figure **3** illustre une variante de réalisation dans laquelle le support ajouré **23** est constitué par un bol **40** dont le bord périphérique **41** est prolongé ou raccordé à une jupe extérieure **42** présentant une hauteur **H** supérieure à la profondeur du bol **40,** de manière à délimiter, entre ce dernier et le fond **2** sur lequel la base de la jupe est destinée à prendre appui, le compartiment **21** décrit précédemment.

L'ensemble bol **40** - jupe périphérique **42** peut être réalisé par conformation et emboutissage d'une plaque perforée, ou par moulage en une matière plastique appropriée, de manière à constituer en quelque sorte une crépine, ou encore par l'association de fils ou tiges liés ensemble.

Il peut aussi être envisagé de ne réaliser la jupe **42** que sous une forme partielle, par exemple au moyen de pattes prolongeant le bord **41**.

La figure **4** représente une deuxième variante de réalisation dans laquelle le support **23** est constitué par une enveloppe **43** en forme de diabolo ou analogue, tubulaire, ouverte aux deux extrémités, ainsi qu'ajourée ou perforée sur sa paroi périphérique. La partie **44** de plus faible section est située à une distance axiale suffisante de l'extrémité basse de l'enveloppe, de façon à délimiter le compartiment **21**, lorsque cette extrémité prend appui sur le fond **2.** La partie **44** peut être entièrement ouverte ou pour partie occupée par une grille ou un croisillon **45** matérialisant le fond du logement **24** qui est délimité par l'enveloppe entre la partie **44** et le bord supérieur **46.**

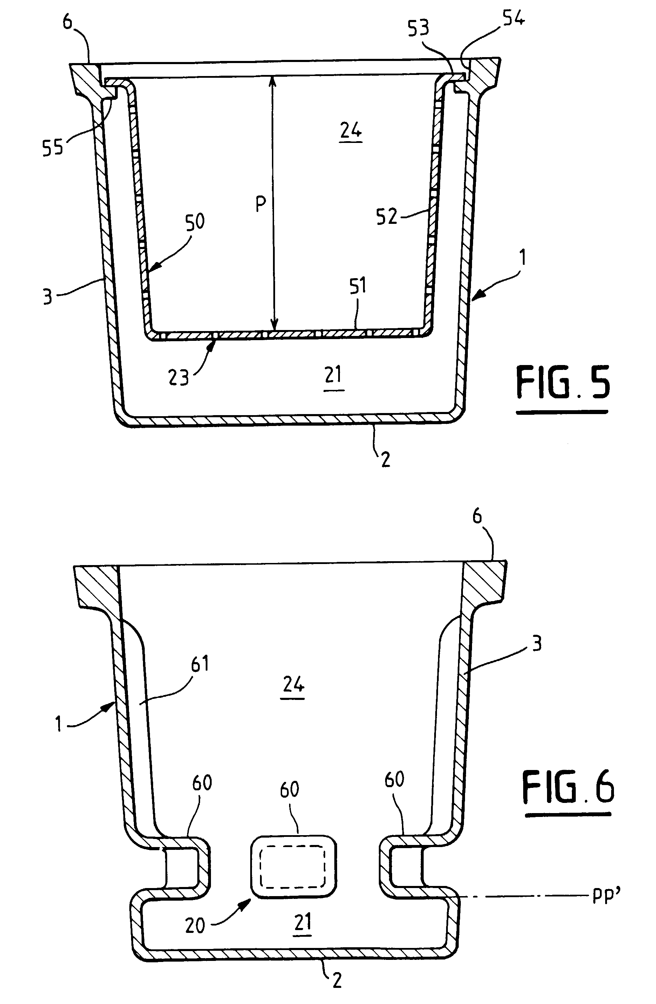

La figure **5** montre une troisième forme de réalisation dans laquelle le support ajouré **23** est réalisé sous la forme d'un bol ou d'une crépine **50** délimitant le logement **24**, possédant un fond ajouré **51** et, dans le plan de l'ouverture de sa paroi périphérique **52**, un rebord **53** orienté vers l'extérieur pour coopérer avec une feuillure **54** délimitée sensiblement en retrait du bord **4**, par exemple par un épaulement **55** de la paroi **3**. Le bol **50** en forme de crépine est alors suspendu par le rebord **53** sur l'épaulement **55** et sa profondeur **P** est déterminée pour que, dans cet état, il délimite, entre son fond **51** et le fond **2** du récipient **1**, le compartiment **21**. Le fond **51** peut dans cet exemple être assimilé aux moyens **20**.

Une autre forme de réalisation est illustrée par la figure **6** dans laquelle le récipient **1** est réalisé de toute manière appropriée et quelque soit sa matière constitutive pour que les moyens **20** soient formés par des conformations rentrantes **60** ménagées périphériquement selon un plan **pp'** situé à distance du fond **2**, de manière à délimiter le compartiment **21.** Les conformations rentrantes 60 délimitent le logement **24** et les moyens prévus pour maintenir à distance de la paroi périphérique **3** la matière ou le matériel **22**, peuvent alors être formés par des nervures **61** de direction axiale, s'étendant vers l'intérieur à partir de la paroi périphérique **3** et, dans la partie de cette dernière, située entre les conformations **60** et le bord supérieur **4**.

L'invention n'est pas limitée aux exemples décrits et représentés car diverses modifications peuvent y être apportées sans sortir de son cadre.

## Revendications

1. Conditionnement de stérilisation et de conservation de matière organique, du type comprenant un récipient **(1)** ou analogue pourvu d'un fond **(2),** des moyens **(20)** établis à distance du fond pour délimiter un compartiment **(21)** au moins en partie ouvert et pour supporter la matière organique, et une coiffe **(7)** à même de fermer le récipient de façon étanche,
caractérisé en ce que le récipient est réalisé en une matière au moins translucide et en ce que les moyens **(20)** établis à distance du fond sont prévus pour
- délimiter un logement **(24)** de réception et de maintien de la matière organique, un tel logement étant ouvert à l'opposé du compartiment **(21)** et communiquant avec ce dernier,
- déterminer au moins un niveau de remplissage du compartiment,
- et maintenir ladite matière à distance de la face interne de la paroi périphérique **(3)** du récipient.

2. Conditionnement selon la revendication **1,** caractérisé en ce que les moyens **(20)** établis à distance du fond **(2)** sont constitués par au moins une conformation rentrante **(60)** présentée par la paroi périphérique du récipient.

3. Conditionnement selon la revendication **1**, caractérisé en ce que les moyens **(20)** établis à distance du fond sont constitués par un support **(23)** ajouré, amovible, disposé à l'intérieur du récipient.

4. Conditionnement selon la revendication **3**, caractérisé en ce que le support **(23)**ajouré est constitué par une sorte de gobelet ou de bol posé sur le fond **(2)**.

5. Conditionnement selon la revendication 3, caractérisé en ce que le support ajouré est constitué par une sorte de crépine (50) suspendue au bord (4) définissant l'ouverture du récipient.

6. Conditionnement selon la revendication **3** ou **4,** caractérisé en ce que le support ajouré est constitué par une enveloppe tubulaire **(43)** en forme de diabolo.

7. Conditionnement selon l'une des revendications **3** à **6**, caractérisé en ce que le support ajouré est pourvu de moyens **(37)** de présentation et de visualisation de témoins fonctionnels.

8. Conditionnement selon l'une des revendications **3** à **7**, caractérisé en ce que le support ajouré est associé à au moins un moyen **(30)** de préhension de la matière organique.

9. Conditionnement selon l'une des revendications **1** à **3** et **5**, caractérisé en ce que les moyens pour déterminer le niveau de remplissage du compartiment **(21)** sont constitués par au moins une graduation **(35)** portée par la paroi du récipient.

10. Conditionnement selon l'une des revendications **1**, **3**, **4** et **6** à **8,** caractérisé en ce que les moyens pour déterminer le niveau de remplissage du compartiment sont formés par des graduations **(36)** portées par le support ajouré.

11. Conditionnement selon la revendication 1, caractérisé en ce que le bord **(4)** du récipient définit une portée annulaire plane **(6)** apte à coopérer avec un joint **(12)** à lèvres concentriques porté par la coiffe **(7)**.

12. Conditionnement selon la revendication **1** ou **11**, caractérisé en ce que la coiffe **(7)** est réalisée en une matière perforable et porte sur sa face extérieure un perforateur **(9)** actionnable manuellement.
